(19) Europäisches Patentamt
European Patent Office
Office européen des brevets

(11) Publication number: 0 129 032
A2

# (12) EUROPEAN PATENT APPLICATION

(21) Application number: 84105034.7

(22) Date of filing: 04.05.84

(51) Int. Cl.³: A 23 L 1/30
A 61 K 37/50, A 61 K 35/78
//A61K31/375, A24B15/30,
C12H1/14

(30) Priority: 13.06.83 JP 105609/83
01.09.83 JP 160880/83

(43) Date of publication of application:
27.12.84 Bulletin 84/52

(84) Designated Contracting States:
AT BE CH DE FR GB IT LI LU NL SE

(71) Applicant: Suntory Limited
1-40 Dojimahama 2-chome Kita-ku
Osaka-shi Osaka-fu 530(JP)

(72) Inventor: Suwa, Yoshihide
7-16 Yamatedai 6-chome
Ibaraki-shi Osaka(JP)

(72) Inventor: Kobayashi, Takumi
1-10 Himuro-cho-1-chome
Takatsuki-shi Osaka(JP)

(72) Inventor: Kiyota, Noriko
14-4 Aoyamadai 4-chome
Suita-shi Osaka(JP)

(72) Inventor: Yoshizumi, Hajime
6-1-612 Kosobe-cho 2-chome
Takatsuki-shi Osaka(JP)

(74) Representative: Hansen, Bernd, Dr.rer.nat. et al,
Hoffmann, Eitle & Partner Patentanwälte Arabellastrasse
4
D-8000 München 81(DE)

## (54) A safe preparation of vitamin C.

(57) A germ component selected from the group consisting of barley malt, wheat germ, soy bean germ, rice germ and extracts thereof reduces or completely inactinates the mutagenicity which is caused or enhanced by the combination of synthetic vitamin C and beverages, tobacco or cupreous ions. Catalase reduces or completely inactivates the mutagenicity and cytotoxicity caused or enhanced by said combination. Therefore, this mutagenicity and cytotoxicity may be reduced or inactivated by preparing a preparation of vitamin C comprising vitamin C and said germ component or catalase.

EP 0 129 032 A2

40 239 u/wa
0129032

# A SAFE PREPARATION OF VITAMIN C

This invention relates to a preparation of vitamin C combining vitamin C with a germ component or catalase [Catalase (hydrogen-peroxide: hydrogen-peroxide oxdoreductase, EC 1. 11. 1. 6)] to reduce or inactivate the mutagenicity of beverages or tobacco caused or enhanced by vitamin C, or to reduce or inactivate the mutagenicity and cytotoxicity of beverages or tobacco coused of enhanced by vitamin C (also known as L-ascorbic acid).

Vitamin C has long been used as a soverign remedy against scurvy. It is also known to be efficacious against viral or bacteriogenous infections diseases such as common cold, influenze, virus pneumonia, hepatitic poliomyelitis, measles, mumps, chickenpox and tuberculosis.

Recently, vitamin C has been found to have the ability to check the growth of malignant tumors (H. Kawasaki et al., Cancer Letters, Vol. 16, 57-63, 1982) or retard the formation of a nitroso compound (S. S. Mirvish et al., Science, Vol. 177, 65-68, 1972). Therefore, vitamin C is considered to be an important substance for cancer prevention. Furthermore, the effectiveness of a large intake of vitamin C is being claimed by several authorities, among whom a Nobel laureate Dr. Linus Pauling is the most prestigious protagonist (E. Cameron et al., Cancer Research, Vol. 39, 663-681, 1979). Today, a high intake of vitamin C in the form of pills or tablets is an everyday matter for an increasing number of people.

However, vitamin C has a strong reducing nature due to an endiol group in the molecule, and in addition to its useful physiological activities, vitamin C may cause deleterious reactions depending on the type of substances with which it is ingested into the body. As a matter of fact, in the presence of cupreous ions, vitamin C exhibits mutagenicity (H.F. Stich et al., Nature, Vol. 260, 722-724,

1976). Incidentally it is presumed that the above mutagenicity is due to radicals such as monodehydroascorbic acid, superoxide amion radical ($\cdot O_2^-$) and hydroxy radical ($\cdot OH$) but it has not yet been proved by experiments. Furthermore, vitamin C causes chromosome aberration in animal cells (H.F. Stich et al., Fd. Cosmet. Toxicol., Vol. 18, 497-501, 1980), enhances the potency of a carcinogen methylcholanthrene (S. Bamit, Cancer Letters, Vol. 11, 239-242, 1981) or works as a cancer-inducing promoter (K. Imaida et al., Proc. Jap. Cancer Association, 41th Ann. Meeting, p. 79, 1982).

It is already known that when vitamin C is dissolved in coffee, the mutagenicity of the resulting solution is at least 6 times as high as that of the coffee alone (Y. Suwa et al., Mutation Res., Vol. 102, 383-391, 1982). According to the studies of the present inventors, the mutagenicity of other beverages such as black tea and bourbon whiskey, as well as tobacco, when ingested together with vitamin C, is increased to a level that is several times as high as that of these beverages or tobacco per se. Since a large intake of vitamin C by an individual is not unusual these days, the development of a method for utilizing only the meritorious effects of vitamin C without endangering the human body will be an urgent matter for the protection of human health.

Since the dawn of its history, man has taken in vitamin C together with other nutrients that occur in nature, and it is quite exceptional in the evolution of animals that vitamin C alone is now ingested in high doses. It is unthinkable that the occurrence of increased mutagenicity due to vitamin C will not cause toxicity in human cells, and when vitamin C is ingested in the form of vegetables and fruits, its deleterious effects are in most likelifood inhibited by some other of the components of vegetables or fruits. Based on this assumption, the present inventors made various studies to find substances that are capable of reducing or completely eliminating the increase in mutagenicity due to vitamin C.

After elaborate screening, the present inventors found that barley malt, wheat germ, rice germ and soy bean germ, and extracts thereof (hereunder referred to as "germ components") have activities which suppress mutagenicity due to the present of vitamin C. Moreover the present inventors also found that catalase has remarkable effects to inactivate the mutagenicity and cytotoxicity caused or increased by the combination of vitamin C and beverages or tobacco.

Catalase has a molecular weight of from 220,000 to 250,000 and is an enzyme decomposing hydrogen peroxide as indicated in the following reaction:

$$2H_2O_2 \xrightarrow{\text{catalase}} 2H_2O + O_2$$

Catalase also acts as a catalyst for oxidation of alcohols, formic acid etc. in the presece of hydrogen peroxide. Catalase is present in most organisms other than anaerobic bacteria and is recognized to be conducive to decomposition or detoxication of hydrogen peroxide endogenously produced in organism living in the aerobic environment.

Any catalases obtained from animal sources including cow and horse livers and erythrocytes; vegetable sources; and microorganisms such as Micrococcus lysodeikticus and Aspergillus niger can be used in this invention.

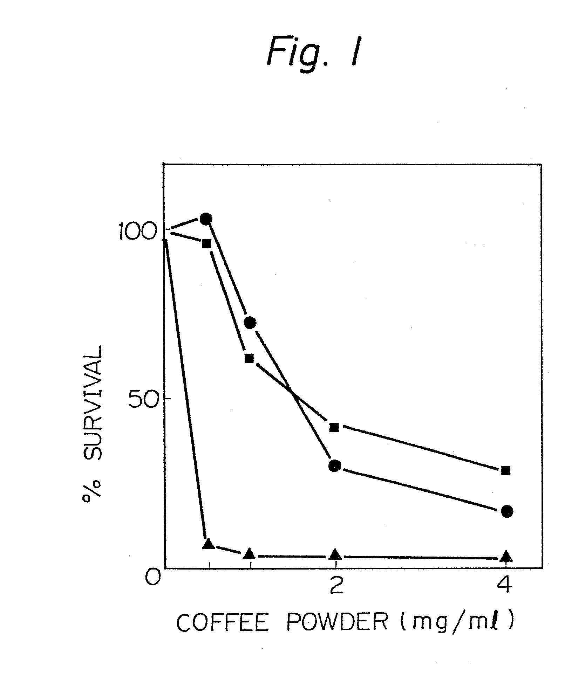

Fig. 1 is a graph indicating the effects of vitamin C in enhancing the cytotoxicity of coffee and the efficacy of catalase in destroying such effects.

In the figure, the symbol ● indicates % survival of coffee per se (containing no additive), the symbol ▲ indicates that of coffee to which vitamin C (0.1 mg/ml) was added and the symbol ■ means % survival of coffee to which vitamin C (0.1 mg/ml) and catalase (10 units/ml) were added.

According to the experiments conducted by the present inventors, vitamin C increases the mutagenicity of coffee by about 6 fold. However, this increase can be suppressed almost completely by mixing vitamin C with a germ powder in an amount not less than five times as much as the vitamin C,

or a germ extract powder in an amount not less than the amount of vitamin C. Further the mutagenicity of coffee per se is also almost completely suppressed. And if 5-25 units of catalase per 1 mg of vitamin C is added, the mutagenicity increase caused by vitamin C can be almost completely suppressed. Similarly, the increase in the mutagenicity of black tea, tobacco or straight bourbon whiskey due to vitamin C can be inhibited in the manner described above.

Consequently, mixing the germ component or catalase with vitamin C and making a preparation that is able to prevent the occurrence of mutagenicity and cytotoxicity owing to the reaction between vitamin C and beverages or tobacco or cupreous ions, without sacrificing the desired activities of vitamin C, is entirely a new use of these substances.

Incidentally the term "combination" or "combining" used herein means any kind of pharmaceutical means able to attain the purposes of this invention, for example, blending of liquid, mixing by using a binder, kneading, combination observed in a multilayer tablet etc.

It will be obvious that a preparation of vitamin C of this invention significantly useful for maintaining human health because the germ component and catalase per se are non-toxic and because the germ agents are rich in vitamins, proteins, essential lipid acids etc. and catalase is an enzyme necessary for protecting an organism against cell lesion by active oxygen.

The preparation of vitamin C of this invention can be used in dosage forms such as oral preparations, e.g., liquid, tables, powder, granules, etc. and the preparation can be used in foods as well as medicines. The preparation containing catalase can also be used in the form of injection.

Substances from which originates the mutagenicity occurring by the combination of vitamin C and beverages, non-alcoholic or alcoholic, or tobacco have not yet been analyzed. As mentioned above, according to many experiments

vitamin C is oxidized to dehydroascorbic acid accompanying the free radicals. But this phenomenon has not yet been directly proved.

First of all, the present inventors investigated the solution of this problem by bringing into focus the reduction of mutagenicity. That is, considering that about 80% of mutagens are carcinogens and also considering that mutagens attack and damage at least DNA constructing genes in cells, it is very important to eliminate or inactivate mutagens for the protection of human health.

The advantages of the present invention will become apparent by reading the following examples to which the invention is by no means limited.

Example I

Efficacy of Germ Components

I) Measuring the mutagenicity and antimutagenic activity

(i) Method: The preincubation method shown in Sugimura & Nagao, Chemical Mutagens, Vol. 6, 41, 1981 was used.

(ii) Microorganism: Histidine requiring *Salmonella typhimurium* TA 100 (hereunder "S.TA 100) was used.

(iii) Preparation of samples

a) Instant coffee: instant coffee powder was disclosed in distilled water. Predetermined amounts of the germ components were respectively suspended or dissolved in distilled water. Each solution was mixed with the aqueous coffee solution to make 100 µl.

b) Bourbon whiskey: Bourbon whiskey was evaporated to dryness under vacuum at a temperature below 40°C in a rotary evaporator. The resulting solid product was dissolved in DMSO. Predetermined amounts of the germ compounds were suspended or dissolved in distilled water. Each solution was mixed with the DMSO solution of the concentrate to make 100 µl.

c) Black tea: Black tea leaves (2.5 g) were brewed with 100 ml of boiling water. The extract was freezedried and the resulting solid matter was dissolved in distilled water to form a solution of a predetermined concentration.

Selected amounts of the germ components were suspended or dissolved in distilled water. Each solution was mixed with the aqueous solution of tea concentrate to make 100 µl.

d) When adding vitamin C to the samples (a)-(c), a solution of vitamin C in 25 µl of distilled water was mixed with each of said samples (25 µl) so as to make the final concentration of vitamin C 0.5 mg/plate. After standing the mixture for 30 minutes at room temperature, it was mixed with the germ component as described hereinafter, suspended or dissolved in distilled water (50 µl), to make 100 µl.

e) The rice germ used was bran manufactured by Gosyo Kabushiki Kaisha (a maker of dried foods in Kobe city, Japan.) The wheat germ used was one commercially available under the trade mark "Riburon" (wheat germ prepared by Nissin Seifun Kabushiki Kaisha in Tokyo, Japan) or one manufactured by Gosyo Kabushiki Kaisha. The barley malt used was one alightly germinated and the soy bean germ used was soy bean immersed in water.

(iv) Measurement of mutagenicity

To 100 µl of each of the samples prepared in (a) to (c), 500 µl of 100 mM sodium phosphate buffer (pH 7.4) and 100 µl of a culture of S.TA 100 were added. Each of the mixtures was shaken for 20 minutes at 37°C, added to 2.5 ml of a soft agar solution and spread on a minimum glucose agar plate which had been supplemented with 50 µM per plate for causing several dinision After incubation at 37°C for 48 hours, the number of colonies on the plate was counted as revertants.

II) Results

(i) Sanctivation of the mutagenicity caused by mixing vitamin C with coffee

The mutagenicity against S.TA 100 caused by mixing vitamin C with coffee was determined.

The mutagenicity of coffee was increased by about 6 fold upon addition of vitamin C. To take coffee as an example, when coffee having a concentration usual to ordinary persons (i.e. 15 mg of coffee powder per ml x 150 ml) is combined with one commercial vitamin pill (75 mg),

the number of revertant colonies for $10^8$ cells is theoretically 315,000. This value is almost equivalent to the mutagenicity caused by about 65 cigarettes.

On the other hand, various germ components almost completely inhibited the mutagenicity of the combination of vitamin C with coffee as shown in Table 1.

Table 1

S.TA 100 strain

| Sample<br>Coffee (mg/plate) | No. of revertants/plate<br>1.5 | 3.75 | 7.5 | 11.25 |
|---|---|---|---|---|
| coffee (no additive) | 12 | 26 | 105 | 244 |
| coffee + vitamin C (vc) | 125 | 190 | 596 | 1,575 |
| coffee + vc + barley malt powder | 3 | 0 | 11 | 13 |
| coffee + vc + barley malt extract powder | 0 | 5 | 4 | 0 |
| coffee + vc + wheat germ powder | 0 | 21 | 19 | 14 |
| coffee + vc + wheat germ extract powder | 0 | 0 | 0 | 7 |
| coffee + vc + bran powder | 2 | 15 | 23 | 23 |
| coffee + vc + bran extract powder | 7 | 6 | 3 | 18 |
| coffee + vc + soy bean extract powder | 10 | 0 | 4 | 16 |

vitamin C: 500 μg/plate

barley malt powder, wheat germ powder, bran powder: 3 mg/plate

barley malt extract powder, wheat germ extract powder, bran extract powder, soy bean extract powder: 1 mg/plate

(ii) Inactivation of mutagenicity due to mixing vitamin C with straight bourbon whiskey

As shown in Table 2 below, various germ components almost completely suppressed the mutagenicity for S.TA 100 caused by mixing vitamin C with straight bourbon whiskey.

Table 2

| Sample | No. of revertants/plate | | | |
|---|---|---|---|---|
| | bourbon whiskey (ml equivalent/plate) | | | |
| | 0.5 | 1.0 | 2.5 | 5 |
| bourbon whiskey (no additive) | 63 | 101 | 379 | 883 |
| bourbon whiskey + vitamin C (vc) | 321 | 480 | 1,052 | 1,799 |
| bourbon whiskey + vc + barley malt powder | 0 | 19 | 30 | 112 |
| bourbon whiskey + vc + barley malt extract powder | 13 | 22 | 44 | 86 |
| bourbon whiskey + vc + wheat germ extract powder | 15 | 0 | 7 | 43 |
| bourbon whiskey + vc + bran extract powder | 50 | 39 | 103 | 179 |

vitamin C: 500 μg/plate

barley malt powder: 3 mg/plate

barley malt extract powder, wheat germ extract powder, bran extract powder: 1 mg/plate

(iii) Inactivation of mutagenicity due to mixing vitamin C with black tea

Although the mutagenicity of black tea is weak in comparison with that of coffee or bourbon whiskey, it is increased upon addition of vitamin C as shown in Table 3 below. The germ components also remarkably reduced the mutagenicity of black tea.

Table 3

| Sample | No. of revertants/plate<br>black tea (ml equivalent/plate) | | | |
|---|---|---|---|---|
| | 0.1 | 0.25 | 0.5 | 1.0 |
| black tea (no additive) | 9 | 26 | 55 | 108 |
| black tea + vitamin C (vc) | 34 | 97 | 161 | 193 |
| black tea + vc + barley malt extract powder | 0 | 3 | 0 | 19 |
| black tea + vc + wheat germ powder | 1 | 7 | 0 | 2 |
| black tea + vc + wheat germ extract powder | 0 | 10 | 7 | 11 |
| black tea + vc + bran extract powder | 0 | 0 | 15 | 28 |

vitamin C: 500 μg/plate

wheat germ powder: 3 mg/plate

barley malt extract powder, wheat germn extract powder, bran extract powder: 1 mg/plate

The term "ml equivalent" in Tables 2 and 3 means an amount in terms of volume (ml) of bourbon whiskey prior to evaporation in I) b) or black tea prior to lyophilization in I) c).

Example II

Efficacy of Catalase

I) Measuring mutagenicity and the antimutagenic effect

(i) Method: See Example I.

(ii) Microorganism: See Example I.

(iii) Preparation of samples

a) Instant coffee: Instant coffee powder was dissolved in distilled water. Predetermined amounts of catalase were diluted with or dissolved in distilled water, and each solution was mixed with the previously prepared instant coffee solution to make 100 l.

b) Bourbon whiskey: straight bourbon whiskey was evaporated to dryness under vacuum ( ≤ 40°C) in a rotary evaporator. The resulting solid material was dissolved in DMSO. Predetermined amounts of catalase were diluted with

distilled water and each solution was mixed with the DMSO solution of alcoholic concentrate to make 100 µl.

c) Black tea: Black tea leaves (2.5 g) were drewed with 100 ml of boiling water, and the extract was freeze-dried. The resulting solid material was dissolved in distilled water to give a selected concentration. Predetermined amounts of catalase were diluted with distilled water, and each solution was mixed with the solution of tea concentrate to make 100 µl.

d) Tobacco tar: Cigarettes were smoked on a smoking machine, and the collected tobacco tar was dissolved in DMSO. Predetermined amounts of catalase were diluted with distilled water, and 25 µl of each solution was mixed with 75 µl of the DMSO solution of tobacco tar. As a control, a 25% DMSO solution of tobacco tar was used.

e) Catalase: An enzyme purified from calf liver and available from P-L Biochemicals, Inc. was used. Its activity was 150,000 U/ml (20,000 U/mg).

f) Addition of vitamin C: When required, vitamin C was dissolved in 25 µl of distilled water to give a concentration of 0.5 mg/plate, and the solution was mixed with 50 µl each of the samples prepared in a) to d). After standing at toom temperature for 30 minutes, each mixture was further combined with 25 µl of a suspension or solution of catalase in distilled water to make 100 µl.

(iv) Measurement of mutagenicity: See Example I.

The percent inhibition of mutagenicity can be calculated by the following formula, if necessary:

$$\text{Percent inhibition} = (1 - \frac{\text{Number of colonies in catalase containing plate}}{\text{Number of colonies in untreated plate}} \times 100\ (\%)$$

(v) Measurement of cytotoxicity

Chinese hamster lung fibroblasts or CHL cells ($5 \times 10^4$) were transferred onto a VAMEM or MEM medium supplemented with vitamins, amino acids and 10% fetal bovine serum, and incubation was made at 37°C for 48

hours in 5% $CO_2$ atmosphere. A flat-bottom tube having a cross-sectional area of 5.5 $cm^2$ was used as an incubator. The medium was treated for 3 hours with 1 ml of VAMEM containing 10% fetal bovine serum with predetermined concentrations of coffee. After the treatment with coffee, the CHL cells were washed once with VAMEM and twice with a phosphate buffer (135 mM NaCl, 2.7 mM KCl, 5.3 mM $Na_2HPO_4$ and 1.45 mM $KH_2PO_4$). The cells were removed by treatment with trypsin, and 200 to 400 cells of them were transferred to petri dish (60 $mm^{\phi}$) and incubated in 5 ml of VAMEM containing 10% fetal bovine embryonic serum. The plating efficiency was checked on the 7th day of the incubation. The percent survival of the cells was determined by the following formula:

$$\text{Percent survival} = \frac{\text{Plating efficiency of treated cells}}{\text{Plating efficiency of untreated cells}} \times 100\ (\%)$$

II) Results

(i) Mutagenicity due to the mixing of vitamin C

The mutagenicity of each of instant coffee, black tea, straight bourbon whiskey and tobacco tar with respect to S.TA 100 was increased by the mixing of vitamin C as shown in Table 4.

Table 4

S.TA 100; without S9 mix

| Sample | No. of revertants/plate | |
|---|---|---|
| | unmixed | mixed with vitamin C (0.5 mg/ml) |
| instant coffee (150 ml) | 43,543 | 315,000 |
| black tea (150 ml) | 16,500 | 60,750 |
| straight bourton whiskey (30 ml) | 5,802 | 22,404 |
| tobacco tar | 4,800 | 15,360 |

The figures in the rightmost column are about 3 to 7 times as great as those in the center column. This value is almost equivalent to the mutagenicity caused by about 65

cigarettes as mentioned in Example I. However, no increase occurred in the mutagenicity of black tea even when it was mixed with lemon juice having a vitamin C content equivalent to that of one commercial vitamin C pill. The above data suggests the following: the mutagenicity of a beverage or tobacco is increased if it is mixed with synthetic vitamin C alone, but as shown by the example of lemon juice, naturally occurring products that contain vitamin C seem to harbor a certain substance that interferes with the reaction causing the increased mutagenicity.

(ii) Inactivation of the mutagenicity due to the mixing with vitamin C

As Table 5 shows, the advantages of catalase are not limited to its ability to reduce the increase in mutagenicity owing to the reaction between vitamin C and coffee, black tea, bourbon whiskey or tobacco; catalase is also effective in reducing or even eliminating the mutagenicity of coffee per se with respect to S.TA 100. For achieving this effect, it would be necessary to use catalase in an amount which is at least equivalent to 10 U.

Table 5

S.TA 100; without S9 mix

| Sample | No. of revertants/plate | | | |
|---|---|---|---|---|
| Catalase (U/plate) | 0 | 1 | 3 | 10 |
| coffee | 244 | 157 | 42 | 0 |
| coffee + vitamin C | 1,575 | 755 | 241 | 8 |
| black tea | 55 | 59 | 48 | 41 |
| black tea + vitamin C | 161 | 123 | 67 | 50 |
| straight bourbon whiskey(SB) | 379 | 362 | 373 | 362 |
| SB + vitamin C | 1,052 | 778 | 425 | 354 |
| tobacco tar(CSC) | 36 | 39 | 43 | 34 |
| CSC + vitamin C | 115 | 90 | 72 | 41 |

The respective samples and vitamin C were used in the following amounts.

Coffee: 11.25 mg/plate

Black tea: 0.5 ml equivalent/plate

Straight bourbon whiskey: 2.5 ml equivalent/plate

Tobacco tar: 0.25 mg/plate

Vitamin C: 0.5 mg/plate (0.35 mg/plate for addition to tobacco tar)

(iii) Inactivation of the cytotoxicity due to the mixing with vitamin C

According to the method shown in I) (V), untreated coffee, one sample containing vitamin C, and one sample having both vitamin C and catalase, were checked for their cytotoxicity against CHL cells. The results are shown in Fig. 1, from which one can see that an appreciably high level of cytotoxicity occurred when vitamin C (0.1 mg/ml) was added to coffee, but this could be completely prevented by further adding catalase (10 U/ml).

(iv) Formulations

<u>Formulation 1</u>

| Components | Amount |
|---|---|
| Ascorbic acid | 2,000 mg |
| Lactose | 6,000 mg |
| Corn starch | 3,000 mg |
| Potato starch | 150 mg |
| Catalase | 20,000 U |

<u>Formulation 2</u>

| | |
|---|---|
| Ascorbic acid | 127.5 g |
| Sodium ascorbate | 127.5 g |
| Powdered sugar (sucrose) | 615.0 g |
| Corn starch | 120.0 g |
| Pigment (sunset yellow) | 50 mg |
| Catalase | 2,000,000 U |

For each formulation, the powder mix of the respective components was directly compressed into tablets. The catalase was a product of Nagase Sangyo K.K. which was commercially used as a food additive (1,000,000 U/g). Magnesium stearate was used as a lubricant.

Claims:

1. A preparation of vitamin C characterized by combining vitamin C with a component selected from the group consisting of catalase and a germ component selected from barley malt, wheat germ, rice germ, soy bean germ and extracts thereof.

2. The preparation according to Claim 1 wherein the component is barley malt or extract thereof.

3. The preparation according to Claim 1 wherein the component is wheat germ or extract thereof.

4. The preparation according to Claim 1 wherein the component is rice germ or extract thereof.

5. The preparation according to Claim 1 wherein the component is soy bean germ or extract thereof.

6. The preparation according to Claim 1 wherein the component is catalase.

7. The preparation according to Claim 6 wherein the proportion of catalase is not less than 5 units per 1 mg of vitamin C.

Fig. I